# EUROPÄISCHE PATENTSCHRIFT

(11) **EP 1 952 772 B1**
(45) Veröffentlichungstag und Bekanntmachung des Hinweises auf die Patenterteilung: **18.08.2010**
(21) Anmeldenummer: 08001034.1
(22) Anmeldetag: 21.01.2008
(51) Int. Cl.: A61B 17/32, A61B 17/22

(54) **Instrument zum operativen Entfernen einer defekten Herzklappe**
Instrument for removing a defective heart valve during an operation
Instrument de retrait opératoire d'une valvule cardiaque défectueuse

(30) Priorität: 01.02.2007 DE 102007005900
(43) Veröffentlichungstag der Anmeldung: 06.08.2008
(73) Patentinhaber: Endosmart Gesellschaft für innovative Medizintechnik mbH, 76297 Stutensee (DE)
(72) Erfinder: Fischer, Harald, Dr. Ing., 76356 Weingarten (DE); Hauck, Florian, Dipl.-Ing., 76139 Karlsruhe (DE)
(74) Vertreter: Pietruk, Claus Peter

(56) Entgegenhaltungen:
- WO-A-2004/089250
- US-A1- 2005 131 438
- US-B1- 6 830 584

## Beschreibung

Die Erfindung betrifft ein instrument zum operativen Entfernen einer defekten Herzklappe. Solche Instrumente werden vorrangig in Alleinstellung, d.h. nur zum Entfernen der defekten Herzklappe verwendet, aber es sind Instrumente dieser Art auch unter Einschluß zusätzlicher Funktionen bekannt, die es z.B. ermöglichen, nach dem Entfernen der defekten Herzklappe mit demselben Instrument und in demselben Operationsvorgang eine neue Herzklappe einzusetzen (vergl. DE 600 17 189 T2, dort Fig. 1, Bezugsziffer 10 mit der dazugehörigen Beschreibung).

Weiterhin ist bekannt, Instrumente dieser Art z.B. mit Auffangvorrichtungen und/oder mit aufspannbaren Filterschirmen zu versehen, um die beim operativen Entfernen einer defekten (und meistens auch verkalkten) Herzklappe frei werdenden Gewebeteile und/oder Materialablagerungen nicht in die Blutbahn des Patienten gelangen zu lassen (vergl. die vorgenannte DE 600 17 169 T2, dort Fig. 1, Bezugsziffer 14 mit der dazugehörigen Beschreibung).

Alle diese Ausführungsformen der Instrumente beinhalten als Grundfunktion immer das Entfernen einer defekten Herzklappe, so daß die nachfolgend beschriebene Erfindung für diese Instrumente grundsätzlich anwendbar ist.

Auch ist die nachfolgend beschriebene Erfindung unabhängig von der jeweiligen Operationsmethode, mittels der das Instrument mit seinem vorlaufenden (distalen) Ende an die defekte Herzklappe herangeführt wird. Das gilt sowohl für die konventionellen Operationsmethoden, die das öffnen des Brustkorbes und das Freilegen eines Zugangs zum Herzen voraussetzen, als auch für die Operationstechniken, die unter Einsatz von Kathetern und Endoskopen eine Herzklappenoperation mit weit geringeren Belastungen für den Patienten ermöglichen (sogenannte minimal-invasive Methoden).

Als minimal-invasive Operationsmethode, bei der ein Instrument gemäß der Erfindung einsetzbar ist, ist auch ein Verfahren bekannt, bei dem zwischen den Rippen eines ungeöffneten Brustkorbes ein Trokar als Instrumentenkanal direkt an das Herz herangeführt und durch die muskuläre Herzwandung hindurch bis an die defekte Herzklappe vorgeschoben wird, so daß über den Trokar am schlagenden Herzen "of pump" (d.h. ohne den Anschluß einer Herz-Lungen-Maschine) operiert werden kann (vergl. hierzu US-PS 5 924 424).

Ein Instrument zum operativen Entfernen einer defekten Herzklappe aus der US 2005/0075659 A1 bekannt, vergleiche insbesondere die dortigen Fig. 2A bis 2C mit der zugehörigen Beschreibung auf Seite 2 ab Ziffer [0037]. Dieses Dokument offenbart konkret in der Fig. 2A ein Instrument mit den Merkmalen des Oberbegriffs des vorliegenden Schutzrechtes; auf die WO 2004/089250 A1, die zur gleichen Patentfamilie wie die US 2005/0075659 A1 gehört, sei ebenfalls hingewiesen.

Minimalinvasive Herzchirurgieinstrumente sind auch bekannt aus der US 6 830 584 B1 sowie aus der US 2005/0131438 A1.

Das bekannte Instrument besitzt zwei an oder auf einem Führungsstab in einem axialen Abstand zueinander angeordnete Werkzeughälften, die jeweils in der Form eines um die Werkzeugachse spiralförmig aufgewickelten Schneidblattes ausgebildet sind. Die Werkzeughälften sind mittels des Führungsstabes relativ zueinander axial verschiebbar derart, daß ihre gegeneinander gerichteten spiralförmigen Schneidblätter eine zwischen den Werkzeughälften positionierte defekte Herzklappe ausschneiden.

Zum operativen Einführen des Instrumentes in das Operationsgebiet sind die spiralförmig aufgewickelten Schneidblätter radial einziehbar, d.h., die in ihrer Ausgangsform etwas loser aufgewickelten Schneidblätter werden strammer gewickelt, wodurch sich der radiale Abstand der Außenwicklung von der Werkzeugachse verkleinert und eine im Durchmesser enger an den Führungsstab angepaßte Berührungsschutzhülse auf die Werkzeughälften aufschiebbar ist. Das hat den prinzipiellen (= gattungsgemäßen) Vorteil, daß das vorlaufende (distale) Ende des Instrumentes mit der enger angepaßten Berührungsschutzhülse relativ problemlos ein stückweit durch die defekte Herzklappe hindurchzuschieben ist bis in eine Position, in der sich die eine Werkzeughälfte auf der einen Seite der defekten Herzklappe und die andere Werkzeughälfte auf der anderen Seite der defekten Herzklappe befindet. Sodann wird in dieser Position des Instrumentes die Berührungsschutzhülse axial von den Werkzeughälften abgezogen, so daß sich deren spiralförmig aufgewickelte Schneidblätter entspannen können. Dadurch vergrößern sich die radialen Abstände der Spiralwicklungen von der Werkzeugachse bis für die jeweilige Außenwicklung des Schneidblattes ein Durchmesser erreicht ist, der dem Durchmesser der auszuschneidenden defekten Herzklappe und dem Durchmesser der nachfolgend operativ einzusetzenden neuen Herzklappe entspricht.

Die beiden Werkzeughälften werden sodann mittels des Führungsstabes (an dem die eine Werkzeughälfte befestigt ist) und mittels eines auf dem Führungsstab geführten Schieberohres (an dem die andere Werkzeughälfte befestigt ist) aufeinander zubewegt und schneiden die defekte Herzklappe aus. Dabei können die Werkzeughälften zur Unterstützung des Schneidvorgangs relativ zueinander rotiert werden.

Nach dem Ausschneiden der defekten Herzklappe folgt die radiale Rückführung der spiralförmigen Schneidblätter auf ihre kleineren Durchmesser und das erneute Aufschieben der Berührungsschutzhülse auf die beiden Werkzeughälften, und zwar bevor das Instrument aus dem Operationsgebiet entfernt wird. Beim erneuten Aufschieben der Berührungsschutzhülse auf die beiden Werkzeughälften können Probleme auftreten, da trotz identisch manueller Handhabungen spiralförmig aufzuwickelnde Schneidblätter unterschiedliche Spiralkonfigurationen und somit unterschiedliche Außendurchmesser aufweisen können. Auf diesen Nachteil der bekannten Instrumente ist in der US-PS 5 924 424 hingewiesen (dort Seite 3 unter Ziffer [0041]).

Eine weitere Störung in der Funktionalität des vorgenannten Instrumentes ergibt sich daraus, daß die spiralförmig aufgewickelten Schneidblätter keinen geometrisch kreisrunden Schnitt erzeugen, wodurch das nachfolgende operative Einsetzen einer neuen Herzklappe erheblich erschwert ist, zumal da derzeit alle mechanisch hergestellten Herzklappenprothesen eine kreisrunde Befestigungsbasis aufweisen.

Auch ist es ein erheblicher Nachteil, daß die spiralförmig gewickelten Schneidblätter der bekannten Instrumente dieser Art eine defekte Herzklappe nicht als einteiliges Klappenstück herausschneiden, sondern das Klappenstück zwischen ihren Spiral-Schneiden primär "zerspanen". Dadurch entstehen zusätzliche Probleme und Notwendigkeiten hinsichtlich der Anordnung von Auffangvorrichtungen und/oder Filterschirmen, um zu verhindern, daß die "zerspanten" Gewebeteile in die Blutbahn des Patienten gelangen.

Die Aufgabe der vorliegenden Erfindung besteht darin, die vorstehend beschriebenen gattungsgemäßen Instrumente so weiterzuentwickeln, daß sie einerseits die operativen Vorteile beibehalten, die sich aus den radial einziehbaren und radial ausfahrbaren Schneidwerkzeugen sowie aus der Verwendung von Berührungsschutzhülsen ergeben, daß sie andererseits aber auch gewährleisten sollen, daß die vorgenannten Nachteile vermieden sind, d.h., daß keine Probleme während der Operation beim erneuten Aufschieben der Berührungsschutzhülsen auf die Werkzeughälften auftreten, daß keine Störungen der Funktionalität des Instrumentes durch unerwünschte "Zerspanungen" der defekten Herzklappe eintreten und daß letztlich auch die Erzeugung eines maßhaltigen kreisrunden Schnittes beim Heraustrennen der defekten Herzklappe gewährleistet ist, damit das nachfolgende operative Einsetzen und Befestigen einer neuen Herzklappe problemlos möglich ist.

Diese Aufgabe wird erfindungsgemäß durch ein Instrument nach Anspruch 1. Vorteilhafte Ausführungsbeispiele sind in den abhängigen Ansprüchen definiert.

Im Gebrauchszustand der beiden Werkzeughälften (im Gebrauchszustand ist der Schneidring der jeweiligen Werkzeughälfte in radialer Richtung voll ausgefahren und maßhaltig auf einen Kreis eingestellt) nutzt die Erfindungsidee die Formstabilität eines flachen Materialbandes, das fertigungstechnisch im Hochkantformat auf einen Halbkreisbogen vorgeformt ist. Diese Vorverformung ist eine im Materialgefüge verfestigte Verformung und somit maßhaltig. Unterstützt wird die Formstabilität durch die Fixierung der beiden Enden des jeweiligen Halbkreisbogens in den vorgenannten Klappachsen, die ihrerseits jeweils durch das Kopfende einer Laschenverbindung fixiert und in axialer Richtung zum Führungsstab des Instrumentes festgelegt sind. Zusätzlich ist auch die jeweilige Mitte der Halbkreisbogen durch eine weitere Laschenverbindung zum Führungsstab des Instrumentes axial festgelegt, so daß die axiale Position und die erforderliche Maßhaltigkeit der Schneidringdurchmesser der Werkzeughälften auch mittels dieser Laschenverbindungen gewährleistet ist.

Für die konstruktiven Ausführungsformen der Schneidringe der Werkzeughälften liegt es im fachlichen Können der Werkzeugbauer, den Schneidringen durch spezielle Einprägungen im Flachbandmaterial (z.B. durch Längs- und/oder Quersicken) eine besondere Formstabilität und Formmaßhaltigkeit zu geben. Im Rahmen des fachlichen Könnens der Werkzeugbauer liegt es auch, eine Auswahl bestimmter Materialeigenschaften zu treffen und/oder die Wahl bestimmter Abmessungen des Hochkantformats, d.h. des Hochkantquerschnitts des zu verarbeitenden Flachbandmaterials zu entscheiden.

Die Erfindung beinhaltet weiterhin die Lehre, wie die im Gebrauchszustand formstabilen Schneidringe der jeweiligen Werkzeughälften auf einen geringeren radialen Abstand zum Führungsstab des Instrumentes einzuziehen sind. Wesentlich hierfür ist zunächst, daß sowohl die Schneidringhälften als auch die Laschenverbindungen aus einem Flachbandmaterial gefertigt sind, das trotz der erforderlichen Formstabilität sehr gute elastische Eigenschaften besitzt (wie z.B. superelastisches Nitinol). Der Einziehvorgang der Schneidringhälften erfolgt durch ein Zusammenspiel der Laschenverbindungen. Gemäß Anspruch 3 sieht eine bevorzugte Ausführungsform der Erfindung vor, daß die Laschenverbindungen, deren Kopfenden an der Mitte der Schneidringhälften angelenkt sind, mit ihren jeweiligen Fußenden am Führungsstab ortsfest gelagert sind und daß die Laschenverbindungen, deren Kopfenden die Kippachsen fixieren, mit ihren jeweiligen Fußenden an oder auf dem Führungsstab axial verschiebbar und arretierbar gelagert sind.

Die vorgenannten Lager-Ausführungen der jeweiligen Fußenden der Laschenverbindungen bestimmen beim Einziehvorgang der Schneidringhälften einen Funktionsablauf, der dadurch gekennzeichnet ist, daß die Laschenverbindungen, die die jeweilige Mitte der Halbkreisbogen der Schneidringhälften fixieren, in ihrer axialen Position verbleiben, wohingegen die Laschenverbindungen, deren Kopfenden mit den Klappachsen des Schneidringes verbunden sind, axial derart verschiebbar und arretierbar sind, daß sie ein Einklappen des Schneidringes um seine Klappachsen bewirken und gleichzeitig die Schneidringhälften veranlassen, sich um ihre in der jeweiligen axialen Position gehaltenen Mitten zu einer Halbform eines schmalen Ovals o.dergl. zu verformen.

Gleichwohl muß hier erwähnt sein, daß die jeweiligen fußseitigen Lager-Ausführungen der Laschenverbindungen auch anders gestaltet sein können, ohne daß dadurch der Rahmen der Lehre der vorliegenden Erfindung verlassen wird. Beispielsweise können auch die Fußenden der Laschenverbindungen, die die jeweilige Mitte der Schneidringhäfften in Position halten, mittels eines Gleitsteins am Führungsstab des Instrumentes verschiebbar und arretierbar gelagert sein. Wenn zudem auch die Fußenden der Laschenverbindungen, die die Kippachsen des Schneidringes fixieren, mittels eines Gleitsteins axial verschiebbar und arretierbar am Führungsstab gelagert sind, dann ergeben sich Funktionsabläufe beim Einziehvorgang der Schneidringhälften, bei denen die jeweils erforderlichen axialen Verschiebungswege auf alle Laschenverbindungen verteilt sind und somit pro Laschenverbindung verkürzt sind.

Auch wäre eine Ausführungsform der Lager-Ausführungen der jeweiligen Fußenden der Laschenverbindungen möglich, bei der die in Anspruch 3 beanspruchte Ausführungsform in ihrer kinematischen Umkehrung realisiert ist, d.h. die Fußenden der den Klappachsen zugeordneten Laschenverbindungen wären am Führungsstab ortsfest zu lagern und die an der Mitte der Schneidringhälften angelenkten Laschenverbindungen wären axial verschiebbar und arretierbar zu lagern. Das Einklappen der Schneidringhälften erfolgt dann dadurch, daß die Klappachsen der Schneidringe in ihrer axialen Position gehalten werden und daß die Mitten der Schneidringhälften von den Klappachsen hinwegbewegt werden.

Für alle diese möglichen Ausführungsformen der Lagerung der fußseitigen Enden der Laschenverbindungen gilt, daß die im Regelfall durch Gleitsteine an oder auf dem Führungsstab ermöglichten Axialverschiebungen mittels einer Schub- und Druckverbindungsstange oder mittels koaxialer Innen- oder Außenhülsen des Führungsstabes erreicht werden, die sich bis zum Handgriff des Instrumentes erstrecken und dort manuell betätigbar sind. Das ist eine für chirurgische Instrumente übliche Handhabung.

Die Laschenverbindungen konfigurieren sowohl im ausgefahrenen Zustand des Schneidringes als auch im eingefahrenen Zustand der Schneidringhälften Meridiane eines trichterförmigen Körpers, der in idealer Weise dafür genutzt werden kann, eine in ihrem Durchmesser enger an den Führungsstab angepaßte Berührungsschutzhülse auf die Schneiden der eingeklappten Schneidringhälften aufzuschieben. Die weichen Übergänge dieser Trichterform und das in radialer Richtung elastische Nachgeben der Laschenverbindungen gewährleisten, daß eine Berührungsschutzhülse jederzeit (und während einer Operation auch wiederholt) auf die eingeklappten Werkzeugschneiden des Instrumentes aufgeschoben und/oder wieder abgezogen werden kann.

Eine besonders vorteilhafte Ausführungsform der Erfindung sieht nach Anspruch 4 vor, daß das Instrument für jede Werkzeughälfte eine eigene Berührungsschutzhülse besitzt, die auf den Laschenverbindungen, die der jeweiligen Werkzeughälfte zugeordnet sind, aufgelagert ist und die auf diesen Laschenverbindungen axial und gegenläufig zu der Berührungsschutzhülse der anderen Werkzeughälfte verschiebbar ist.

Weitere Verbesserungen des erfindungsgemäßen Instrumentes sind gemäß Anspruch 5 in der Weise möglich, daß der Durchmesser des Schneidringes der einen Werkzeughälfte geringfügig größer ist als der Durchmesser des Schneidringes der anderen Werkzeughälfte derart, daß beide Schneidringe beim Ausschneiden der zwischen ihnen positionierten defekten Herzklappe unter Bildung eines engen Scherspaltes ineinandergreifen.

Gemäß Anspruch 6 kann weiterhin vorgesehen sein, daß die Werkzeughälften mit ihren Schneidringen relativ zueinander rotierbar sind derart, daß die defekte Herzklappe durch einen Dreh-Stanz-Vorgang ausgeschnitten wird.

Nachfolgend wird ein Ausführungsbeispiel der Erfindungen anhand der Zeichnungen näher beschrieben. Es zeigen:
- Fig. 1 und 2: in vereinfachter Darstellung die konstruk- tiven Funktionsteile eines erfindungsgemä- ßen Instrumentes,
- Fig. 3 bis 5: in perspektivischer Darstellung den Ein- ziehvorgang der Schneidringhälften bei ei- nem Instrument gemäß den Figuren 1 und 2.
Der grundsätzliche Aufbau eines erfindungsgemäßen Instrumentes ist am besten aus der Fig. 3 in Verbindung mit den Figuren 1 + 2 ersichtlich.

Zu erkennen ist der zentrale Führungsstab 10, der sich in den Darstellungen nach unten bis zu einem instrumentalen Handgriff fortsetzt, der in den Figuren nicht mit dargestellt ist, da er von üblicher Konstruktion und Gestaltung sein kann. In und an einem solchen Handgriff sind diverse Stellantriebe und Arretierungsvorrichtungen vorhanden, mittels der die Funktionsteile des Instrumentes, wie sie nachfolgend benannt sind, jeweils einzeln gesteuert (verstellt) und/oder arretiert werden können. Das geschieht üblicherweise mittels zum Führungsstab parallel laufender Steuerstangen und/oder koaxial ineinander geführten Steuerhülsen.

Koaxial um den Führungsstab 10 sind zwei spiegelbildlich zueinander ausgebildete Werkzeughälften 11 und 12 vorhanden, jeweils bestehend aus einem Schneidring 13 und 14 mit ihren jeweils vier Laschenverbindungen 15 bis 18 und 19 bis 22, die jeweils mit ihren Fußende am Führungsstab 10 gelagert sind. Die Schneidringe sind aus einem elastisch verformbaren Flachbandmaterial gefertigt und auf eine geometrisch exakte Kreisform vorgeformt. Die Laschenverbindungen sind in Längsrichtung des Führungsstabes zug- und druckfest, aber in radialer Richtung zum Führungsstab beim Auftreten entsprechender radialer Kraftkomponenten elastisch verformbar.

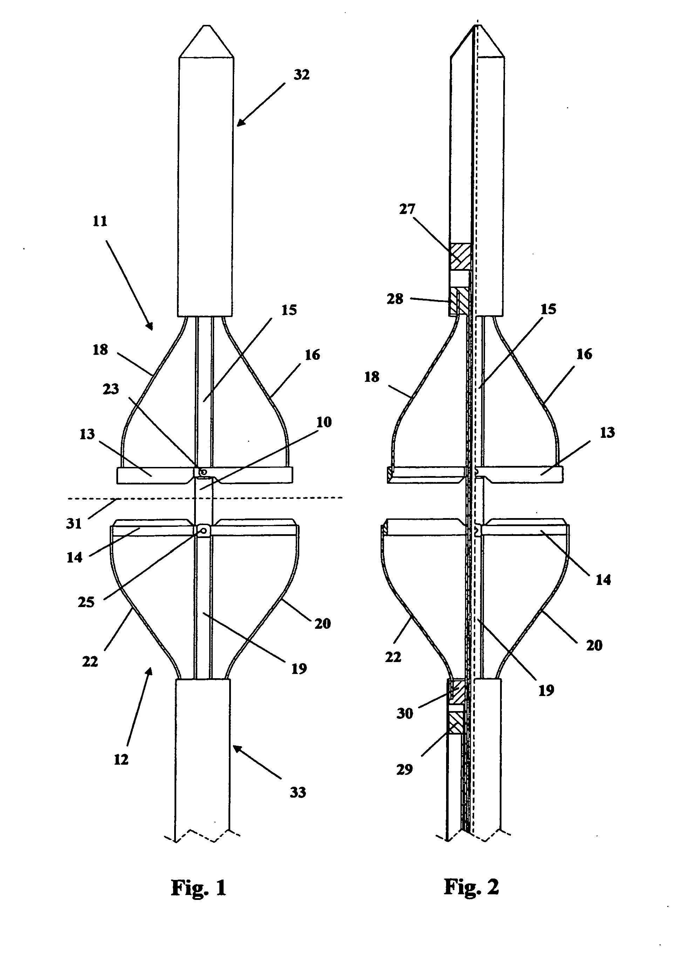

Die bei jeder Werkzeughälfte einander gegenüberliegenden Laschenverbindungen 15 und 17(siehe obere Werkzeughälfte) bzw. 19 und 21 (siehe untere werkzeughälfte) fixieren kopfseitig die Klappachsen 23 und 24 bzw. 25 und 26. Bei der oberen Werkzeughälfte sind die Fußenden der Laschenverbindungen 15 und 17 in einem Gleitstein 27 und die Fußenden der Laschenverbindungen 16 und 18 an einem Lagerstein 28 befestigt (siehe Fig. 2). Bei der unteren Werkzeughälfte gilt das entsprechend für den Gleitstein 29 und den Lagerstein 30 (siehe Fig. 2) .

Die jeweiligen Lagersteine 28 und 30 sind in ihrer axialen Position ortsfest gehalten, wohingegen die Gleitsteine 27 und 29 axial verschiebbar und arretierbar am Führungsstab 10 gelagert sind.

Die obere Werkzeughälfte 11 wird durch eine Rotation des Führungsstabes 10 auf die untere Werkzeughälfte 12 zubewegt, so daß mittels eines Dreh-Stanz-Vorgangs eine defekte Herzklappe, die in der Ebene 31 zwischen den beiden Werkzeughälften positioniert ist (siehe Fig. 1), und durch die unter Bildung eines engen Scherspaltes ineinandergreifenden Schneidringe 13 und 14 ausgeschnitten wird. Die Funktionsbauteile zum Rotieren der oberen Werkzeughälfte 10 sind aus Gründen der besseren Übersichtlichkeit in den Figuren nicht mit dargestellt. Sie sind von üblicher Bauart.

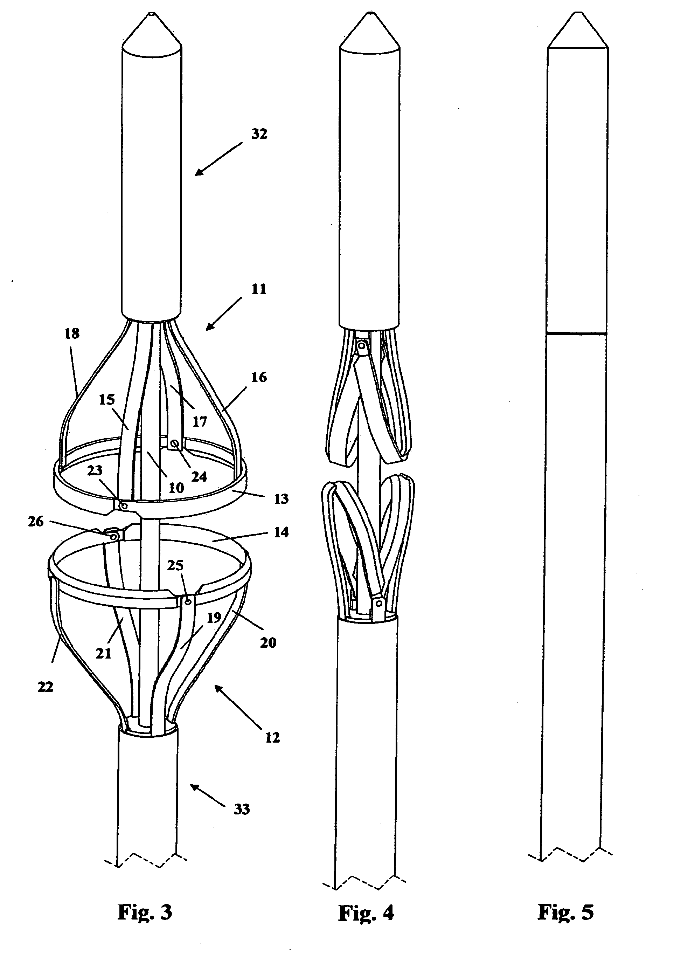

Zum Einklappen der Schneidringe 13 und 14 bzw. deren jeweiligen halbkreisförmigen Schneidringhälften, die miteinander mittels der Klappachsen 23 + 24 bzw. 25 + 26 verbunden sind, werden die den jeweiligen Klappachsen zugeordneten Laschenverbindungen 15 + 17 bzw. 19 + 21 mittels ihrer fußseitigen Gleitsteine 27 bzw. 29 in axialer Längsrichtung des Führungsstabes verschoben, und zwar bei der oberen Werkzeughälfte darstellungsgemäß nach oben und bei der unteren Werkzeughälfte darstellungsgemäß nach unten. Diese Laschenverbindungen nehmen dann die Positionen ein, wie sie aus Fig. 4 ersichtlich sind.

Die anderen Laschenverbindungen 18 +16 der oberen Werkzeughälfte und 20 + 22 der unteren Werkzeughälfte halten ihre jeweiligen axialen Positionen, so daß sich die jeweiligen Schneidringhälften in die Halbform eines Ovals (oder ähnlich) verformen, wie dies in Fig. 4 gezeigt ist.

Im Ergebnis wird sodann ein eingezogener Zustand der Laschenverbindungen und der Schneidringhälften erreicht, bei dem die beiden Berührungsschutzhülsen 32 und 33 jeweils gegenläufig und problemlos auf die eingefahrenen bzw. eingezogenen Werkzeughälften aufgeschoben werden können. Den mittels der Berührungsschutzhülsen vollständig geschlossenen Zustand des erfindungsgemäßen Instrumentes zeigt Fig. 5.

## Patentansprüche

1. Instrument zum operativen Entfernen einer defekten Herzklappe, wobei das Instrument zwei an oder auf einem Führungsstab koaxial und in einem Abstand zueinander angeordnete Werkzeughälften besitzt, die gegeneinander gerichtete Werkzeugschneiden aufweisen und die mittels des Führungsstabs relativ zueinander axial derart verschiebbar sind, dass ihre gegeneinander gerichteten Werkzeugschneiden eine zwischen den Werkzeughälften positionierte, defekte Herzklappe herausschneiden können, wobei die Schneiden der Werkzeughälften zum Einführen des Instrumentes in das Operationsgebiet so einziehbar sind, dass deren radialer Abstand von der Werkzeugachse verkleinert wird und eine im Durchmesser enger an den Führungsstab angepasste Berührungsschutzhülse auf die Werkzeughälften mit den radial eingezogenen Schneiden axial aufschiebbar ist, so dass das vorlaufende Ende des Instruments mit der im Durchmesser enger an den Führungsstab angepassten Berührungsschutzhülse ein Stück weit durch die defekte Herzklappe bis in eine Position hindurchschiebbar ist, in der sich die eine Werkzeughälfte auf der einen Seite der defekten Herzklappe und die andere Werkzeughälfte auf der anderen Seite der defekten Herzklappe befindet, wobei in dieser Position nach axialem Abzug der Berührungsschutzhülsen von den Werkzeughälften die Schneiden der Werkzeughälften so ausfahrbar sind, dass deren radialer Abstand von der Werkzeugachse bis auf einen Durchmesser vergrößert ist, der dem Durchmesser der auszuschneidenden, defekten Herzklappe und dem Durchmesser einer nachfolgend operativ einzusetzenden, neuen Herzklappe entspricht,
**dadurch gekennzeichnet, dass**
die Schneiden der Werkzeughälften jeweils aus zwei halbkreisförmigen Schneidringhälften gebildet sind, die endseitig miteinander über je eine einachsige Gelenkverbindung (23, 24, 25, 26)zu einem kreisförmigen Schneidring (13, 14)verbunden sind, wobei jede Schneidringhälfte aus einem elastisch verformbaren, flachen Materialband gefertigt ist und auf den radial ausgefahrenen, geforderten Halbkreisdurchmesser maßhaltig im Hochkantformat vorgeformt ist,
die Klappachsen der Schneidringhälften senkrecht zur Achse des Führungsstabs (10) ausgerichtet sind und jeweils mittels des Kopfendes einer elastisch verformbaren, zug- und druckfesten Laschenverbindung (15, 17, 19, 21) fixiert sind, die sich in Längsrichtung des Führungsstabs erstrecken und die jeweils mit ihren Fußende am Führungsstab gelagert sind, und
jede Schneidringhälfte mittels einer weiteren, elastisch verformbaren, zug- und druckfesten, sich in Längsrichtung des Führungsstabs erstreckenden und mit ihrem Fußende am Führungsstab gelagerten Laschenverbindung (16, 18, 20, 22) gehalten ist, deren Kopfende in der Mitte des Halbkreises an der im ausgefahrenen Zustand halbkreisförmigen Schneidringhälfte angelenkt ist, wobei diese Anlenkung und/oder die elastische, verformbare Laschenverbindung der Schneidringhälfte erlaubt, eine Schwenkbewegung von nahezu 90° um eine quer zur Laschenverbindung verlaufende Achse auszuführen.

2. Instrument nach dem vorhergehenden Anspruch, **dadurch gekennzeichnet, dass** das flache Materialband aus superelastischem Nitinol besteht.

3. Instrument nach einem der vorhergehenden Ansprüche, **dadurch gekennzeichnet, dass** die Laschenverbindungen (16, 18, 20, 22), deren Kopfenden an der Mitte der Schneidringhälften angelenkt sind, mit ihren jeweiligen Fußenden am Führungsstab (10) ortsfest gelagert sind, und dass die Laschenverbindungen (15, 17, 19, 21), deren Kopfenden die Kippachsen fixieren, mit ihren jeweiligen Fußenden an oder auf dem Führungsstab (10) axial verschiebbar und arretierbar gelagert sind.

4. Instrument nach einem der vorhergehenden Ansprüche, **dadurch gekennzeichnet, dass** das Instrument für jede Werkzeughälfte eine eigene Berührungsschutzhülse (32, 33) besitzt, die auf den Laschenverbindungen, die der jeweiligen Werkzeughälfte zugeordnet sind, aufgelagert ist und die auf diesen Laschenverbindungen axial und gegenläufig zu der Berührungsschutzhülse der anderen Werkzeughälfte verschiebbar ist.

5. Instrument nach einem der vorhergehenden Ansprüche, **dadurch gekennzeichnet, dass** der Durchmesser des Schneidrings (14) der einen Werkzeughälfte (12) geringfügig größer ist als der Durchmesser des Schneidrings (13) der anderen Werkzeughälfte (11) derart, dass beide Schneidringe beim Ausschneiden der zwischen ihnen positionierten defekten Herzklappe unter Bildung eines engen Scherspaltes ineinandergreifen.

6. Instrument nach einem der vorhergehenden Ansprüche, **dadurch gekennzeichnet, dass** die Werkzeughälften mit ihren Schneidringen relativ zueinander derart rotierbar sind, dass die defekte Herzklappe durch einen Dreh-StanzVorgang ausgeschnitten wird.

## Claims

1. Instrument for the surgical removal of a defective heart valve, wherein the instrument has two tool halves, which are arranged coaxially and at a distance from each other at or on a guide rod, have opposing cutting tool edges and are displaceable axially in relation to each other by means of the guide rod in such a way that their opposing cutting tool edges can cut out a defective heart valve positioned between the tool halves, wherein, to introduce the instrument into the operating area, the cutting edges of the tool halves can be retracted in such a way that their radial distance from the tool axis is reduced and a contact guard sleeve, adapted more closely in diameter to the guide rod, can be pushed axially onto the tool halves with the radially retracted cutting edges, so that the leading end of the instrument with the contact guard sleeve adapted more closely in diameter to the guide rod can be pushed a little way through the defective heart valve into a position in which one tool half is on one side of the defective heart valve and the other tool half is on the other side of the defective heart valve, wherein, in this position, after axial withdrawal of the contact guard sleeves from the tool halves, the cutting edges of the tool halves can be extended in such a way that their radial distance from the tool axis is increased to a diameter which corresponds to the diameter of the defective heart valve to be cut out and to the diameter of a new heart valve subsequently to be surgically introduced, **characterized in that** the cutting edges of the tool halves are respectively formed by two semicircular cutting ring halves, which are connected to each other at the ends by respective single-axis hinged connections (23, 24, 25, 26) to form a circular cutting ring (13, 14), wherein each cutting ring half is produced from an elastically deformable, flat material strip and is pre-shaped in edge-wise format to the radially extended, required semicircular diameter in a dimensionally stable manner,
the hinge axes of the cutting ring halves are aligned perpendicularly to the axis of the guide rod (10) and are respectively fixed by means of the top end of an elastically deformable, tension- and compression-resistant butt-strap connection (15, 17, 19, 21), which connections extend in the longitudinal direction of the guide rod and are respectively mounted by their bottom ends at the guide rod, and
each cutting ring half is held by means of a further, elastically deformable, tension- and compression-resistant butt-strap connection (16, 18, 20, 22) extending in the longitudinal direction of the guide rod and mounted with its bottom end at the guide rod, the top end of which is hinged in the middle of the half-circle on the cutting ring half that is semicircular in the extended state, wherein this hinge mounting and/or the elastic, deformable butt-strap connection of the cutting ring half allows a pivoting movement of almost 90° to be performed about an axis running transversely in relation to the butt-strap connection.

2. Instrument according to the preceding claim, **characterized in that** the flat material strip consists of superelastic nitinol.

3. Instrument according to one of the preceding claims, **characterized in that** the butt-strap connections (16, 18, 20, 22) of which the top ends are hinged at the middle of the cutting ring halves are mounted with their respective bottom ends in a fixed manner at the guide rod (10), and **in that** the butt-strap connections (15, 17, 19, 21) of which the top ends fix the tilting axes are mounted with their respective bottom ends in an axially displaceable and arrestable manner at or on the guide rod (10).

4. Instrument according to one of the preceding claims, **characterized in that** the instrument has for each tool half a dedicated contact guard sleeve (32, 33), which is mounted on the butt-strap connections that are assigned to the respective tool half and is displaceable on these butt-strap connections axially and in opposition to the contact guard sleeve of the other tool half.

5. Instrument according to one of the preceding claims, **characterized in that** the diameter of the cutting ring (14) of one tool half (12) is slightly greater than the diameter of the cutting ring (13) of the other tool half (11) in such a way that, when cutting out the defective heart valve positioned between them, the two cutting rings engage in each other to form a narrow shearing gap.

6. Instrument according to one of the preceding claims, **characterized in that** the tool halves are rotatable with their cutting rings in relation to each other in such a way that the defective heart valve is cut out by a turning-punching operation.

## Revendications

1. Instrument de retrait opératoire d'une valvule cardiaque défectueuse, dans lequel l'instrument possède deux moitiés d'outil disposées contre ou sur une barre de guidage coaxialement et à distance l'une de l'autre, qui présentent des tranchants d'outil orientés l'un vers l'autre, et qui peuvent être déplacées axialement au moyen de la barre de guidage relativement l'une par rapport à l'autre de telle sorte que leurs tranchants d'outil orientés l'un vers l'autre puissent couper une valvule cardiaque défectueuse située entre les moitiés d'outil, les tranchants des moitiés d'outil pouvant être vissés pour enfoncer l'instrument dans la zone opératoire, de telle sorte que leur distance radiale à l'axe de l'outil soit réduite et qu'une douille de protection de contact de diamètre étroit, adaptée à la barre de guidage, puisse être poussée axialement sur les moitiés d'outil avec les tranchants vissés radialement, de sorte que l'extrémité avant de l'instrument avec la douille de protection de contact adaptée à la barre de guidage, de diamètre étroit, puisse être enfoncée un peu plus à travers la valvule cardiaque défectueuse jusque dans une position dans laquelle l'une des moitiés d'outil se trouve sur un côté de la valvule cardiaque défectueuse et l'autre moitié d'outil se trouve de l'autre côté de la valvule cardiaque défectueuse, les tranchants des moitiés d'outil, dans cette position, pouvant être sortis après le retrait axial des douilles de protection de contact hors des moitiés d'outil, de telle sorte que leur distance radiale à l'axe de l'outil étant augmentée jusqu'à un diamètre qui correspond au diamètre de la valvule cardiaque défectueuse à couper, et au diamètre d'une nouvelle valvule cardiaque à insérer ensuite par voie opératoire,
**caractérisé en ce que**
les tranchants des moitiés d'outil sont à chaque fois formés de deux moitiés de bague de coupe de forme semi-circulaire, qui sont connectées à leur extrémité l'une à l'autre par le biais d'une connexion articulée à un axe respective (23, 24, 25, 26) pour former une bague de coupe de forme circulaire (13, 14), chaque moitié de bague de coupe se composant d'une bande de matériau plate déformable élastiquement, et étant préformée au diamètre de demi-cercle exigé sorti radialement en respectant les dimensions au format sur chant,
les axes de rabattement des moitiés de bague de coupe sont orientés perpendiculairement à l'axe de la barre de guidage (10) et sont fixés à chaque fois au moyen de l'extrémité de tête d'une connexion à pattes déformable élastiquement, rigide en traction et en pression (15, 17, 19, 21), qui s'étendent dans la direction longitudinale de la barre de guidage et qui sont à chaque fois montées avec leurs extrémités de base sur la barre de guidage, et
chaque moitié de bague de coupe est maintenue au moyen d'une autre connexion à pattes (16, 18, 20, 22) déformable élastiquement, rigide en traction et en pression, s'étendant dans la direction longitudinale de la barre de guidage et montée avec ses extrémités de base sur la barre de guidage, dont l'extrémité de tête est articulée au milieu du demi-cercle à la moitie de bague de coupe en forme de demi-cercle dans l'état sorti, cette articulation et/ou la connexion à pattes élastique déformable des moitiés de bague de coupe permettant d'effectuer un mouvement de pivotement de presque 90° autour d'un axe s'étendant transversalement à la connexion à pattes.

2. Instrument selon la revendication précédente, **caractérisé en ce que** la bande de matériau plate se compose de nitinol super élastique.

3. Instrument selon l'une quelconque des revendications précédentes, **caractérisé en ce que** les connexions à pattes (16, 28, 20, 22), dont les extrémités de tête sont articulées au centre des moitiés de bague de coupe, sont montées fixement avec leurs extrémités de base respectives sur la barre de guidage (10), et **en ce que** les connexions à pattes (15, 17, 19, 21), dont les extrémités de tête fixent les axes de basculement, sont montées avec leurs extrémités de base respectives contre ou sur la barre de guidage (10), de manière déplaçable et blocable axialement.

4. Instrument selon l'une quelconque des revendications précédentes, **caractérisé en ce que** l'instrument possède pour chaque moitié d'outil une douille de protection de contact propre (32, 33), qui est posée sur les connexions à pattes qui sont associées à la moitié d'outil respective, et qui peut être déplacée axialement sur ces connexions à pattes et en sens inverse de la douille de protection de contact de l'autre moitié d'outil.

5. Instrument selon l'une quelconque des revendications précédentes, **caractérisé en ce que** le diamètre de la bague de coupe (14) d'une moitié d'outil (12) est légèrement plus grand que le diamètre de la bague de coupe (13) de l'autre moitié d'outil (11), de telle sorte que les deux bagues de coupe, lors de la coupure de la valvule cardiaque défectueuse positionnée entre elles, viennent en prise l'une dans l'autre en formant une fente de cisaillement étroite.

6. Instrument selon l'une quelconque des revendications précédentes, **caractérisé en ce que** les moitiés d'outil peuvent tourner l'une par rapport à l'autre avec leurs bagues de coupe de telle sorte que la valvule cardiaque défectueuse soit coupée par une opération de rotation-estampage.
